# EUROPEAN PATENT SPECIFICATION

(11) **EP 2 575 678 B1**
(45) Date of publication and mention of the grant of the patent: **16.05.2018**
(21) Application number: 11722095.4
(22) Date of filing: 31.05.2011
(51) Int. Cl.: A61F 2/07

(54) **DEVICE FOR PLACEMENT IN A HOLLOW ORGAN, IN PARTICULAR FOR HOLDING OPEN SAID HOLLOW ORGAN AND METHOD FOR PRODUCING SUCH DEVICE**
VORRICHTUNG ZUR POSITIONIERUNG IN EINEM HOHLORGAN, IM BESONDEREN ZUR BEWAHRUNG DES HOHLORGANS IN GEÖFFNETER POSITION UND VERFAHREN ZUR HERSTELLUNG EINER DERARTIGEN VORRICHTUNG
DISPOSITIF POUR PLACEMENT DANS UN ORGANE CREUX, EN PARTICULIER POUR MAINTENIR OUVERT LEDIT ORGANE CREUX ET PROCÉDÉ POUR PRODUIRE UN TEL DISPOSITIF

(30) Priority: 17.01.2011 US 201161433400 P; 02.06.2010 EP 10164721
(43) Date of publication of application: 10.04.2013
(73) Proprietor: Occlutech Holding AG, 8201 Schaffhausen (CH)
(72) Inventor: WILLEMS, Frank, 50829 Cologne (DE); CLAßEN, Christoph, 50829 Cologne (DE); HENSELER, Andreas, 50829 Cologne (DE); WITT, Wolfgang, 50829 Cologne (DE)
(74) Representative: KIPA AB
(86) International application number: PCT/EP2011/058931
(87) International publication number: WO 2011/151318

(56) References cited:
- DE-A1-102006 028 221
- US-A- 5 549 663
- US-A1- 2003 195 611
- US-A1- 2004 030 377

## Description

The present invention relates to a device for placement in a hollow organ, in particular for holding open the hollow organ, and a method for producing such a device.

In an exemplary application, the device for placement in a hollow organ is a vascular support for holding open blood vessels, which herein will be mentioned as an example of a (biological) hollow organ, which is known *inter alia* under the term "stent". Vascular prostheses, on the other hand - in contrast to vascular supports which serve for holding open e.g. a blood vessel and thus do not replace the blood vessel and respectively the hollow organ - are medical products provided for replacement of biological tissues.

The invention will be explained hereunder, by way of example, with reference to a vascular support for a blood vessel embodied as a placement device for hollow organs, wherein, first, the problematics of presently known vascular supports will be outlined.

The clinical picture of arteriosclerosis is characterized by a pathological deposition of various substances - *inter alia* calcium and fat - on the wall of a blood vessel (arteriosclerotic plaque). The resultant narrowing of the inner vessel diameter causes a reduced passage for the blood flow, which in turn will result in an undersupply to the tissue downstream of this site.

The above narrowing of vessels can be clinically reversed by means of a vascular support (stent), which is effected by mechanically widening the vessel wall and keeping it in this state by placement of the stent. However, after such an intervention, it frequently happens that a renewed narrowing develops at the given site because the vessel wall may proliferate into the vessel lumen through the interspaces of the stent lattice.

In case of an instable plaque, only a small spatial delimitation exists between the substances deposited on the vessel wall and the interior of the vessel (blood flow). In such a case, a plaque rupture may occur so that the deposited substances will enter the blood flow. In the periphery, these substances can cause occlusions or initiate thrombotic occlusions. This risk is further increased by use of non-coated vascular supports In arteriosclerotic vessels covered by instable, inelastic plaque because, at such sites, the dilatation of the vascular support will cause an increased mechanical stress acting on the inner wall of the vessel, which in turn will heighten the risk of rupture.

In a further type of clinical application, vascular supports are used for treatment of aneurysms in vessels. In this case, use is made of special vascular supports whose wall is formed by a closed material, normally, a PTFE of little elasticity. These vascular supports have to be implanted with the aid of a complex applicator in order to allow them to be converted, within the vessel, from a folded state to an expanded state and to be placed in position. This process entails the risk that folds may be generated in cases where it had not been possible to accomplish an optimal adaptation of the vascular support diameter to the vessel diameter.

From DE 102 23 399 B4, there is known a vascular support, provided for supporting a vessel wall, which comprises mutually adjacent support elements and a film of absorbable material enclosing said support elements, wherein said film can consist of a nonwoven. Said film or fleece has the function of keeping the individual support elements at a distance until, over time, they will have become fixed to the vessel wall. Said film or fleece will be absorbed so that, of the overall vascular support, it is only the support elements which will ultimately remain in the vessel.

Known from DE 10 2006 028 221 A1 is a hose-shaped vascular prosthesis comprising a wall with an elastic fleece structure which under physiological conditions is substantially non-absorbable. The vascular prosthesis can be provided with reinforcement elements. As already mentioned above, the vascular prosthesis serves for replacement of biological tissue and not for the purpose of maintaining it in an open state or for the purpose of supporting hollow organs. Due to this intended use, it is not possible in vascular surgery to use prostheses for maintaining hollow organs in an open state because the prostheses, according to their purpose, shall replace the hollow organs and thus, logically, cannot support a hollow organ while the latter remains in place.

Another prosthesis, namely an end prosthesis, is described in US-A-5 549 663. This prosthesis comprises a stent component and a graft component capturing a part of the stent component. Said graft component consists of helically wound fibers by which the thus tubular graft component is given an axial and thus anisotropic and respectively one-dimensional preferred stretching direction.

US2003/0195611 discloses fibers created by an electrospinning process and that have diameters averaging less than 100 micrometers. Proper manipulation of the identified variables ensures that these fibers are still wet upon contacting a target surface, thereby adhering with each other to form a cloth-like material and, if desired, adhering to the target surface to form a covering thereon.

US 2004/0030377 discloses a stent assembly comprising an expensible tubular supporting element and at least one coat of electrospun polymer fibers, each of the at least one coat having a predetermined porosity, the at least one coat including at least one pharmaceutical agent incorporated therein for delivery of the at least one pharmaceutical agent into a body vasculature during or after implantation of the stent assembly within the body vasculature.

It is an object of the invention to provide a device for placement in a hollow organ wherein the risk of the above mentioned complications during and after implantation is reduced.
The invention is defined by the claims.
According to the present invention a device for placement in a hollow organ comprises
- a placement body having an inner side and an outer side, and
- at least one layer of biostable random-fiber fleece material arranged at said placement body and at least partially abutting thereon.

The invention further proposes a method for producing said device wherein, according to said method, there is first provided said placement body and, for positioning of said layer of biostable random-fiber fleece material, the placement body is sprayed with fibers by use of a spraying device while the placement body and the spraying device are moved relative to each other, and wherein, in dependence on the desired thickness of said layer of biostable random-fiber fleece material, a plurality of layers of fibers are sprayed on.

In essence, the placement body for placement in a hollow organ is be provided with a biostable fleece material comprising interconnected and, particularly, fine-fibrillated fibers made e.g. of polyethylene. In this arrangement, the biostable random-fiber fleece material (hereunder referred to merely as a fleece material) is at least partially in abutment on the inner side and/or on the outer side of the placement body of the device.

The following is a description of the invention with reference to its application as a vascular support for holding open a hollow organ, wherein the placement body is referred to as a support body.

Said fleece material, which is biostable, i.e. under physiological conditions is substantially not absorbable, preferably comprises a fine-fibrillated fleece whose fibers are connected to each other. Particularly, this fleece material can have such a porosity that the liquid components of the blood or of the substances taken up by the hollow organ will be substantially allowed to pass while, however, the cellular components of the blood or of the substances taken up by the hollow organ and of the vessel wall will be substantially retained. This feature advantageously allows for a complete exchange of liquids and chemical elements, particularly of nutrients, metabolites and other physiological substances between the inner fluid and the vessel wall. By the random-fiber fleece material of the invention, the support body can be stretched in an isotropic manner, which is of advantage for a multidimensional supporting function that is effective in a plurality of spatial directions.

According to a further embodiment, it is provided that the fleece material layer in the area of the inner surface has a porosity which is smaller than the porosity on the outer surface of the fleece material layer. When viewed in the thickness direction of the fleece material layer, the porosity of the fleece material can vary in a continuous, quasi-continuous or step-wise manner.

According to a further embodiment, the biostable fleece material, on its inner surface which comes into contact with the substances taken up by the hollow organ, is capable of rendering possible, or facilitating, the adherence and colonization of blood cells, stem cells, progenitor cells or blood-vessel wall cells or, more generally, of cells of the substances taken up by the hollow organs. This is achieved particularly by corresponding selection of the porosity of the fleece material on the surface thereof which comes into contact with the tissue, and of the porosity at that site of the fleece material layer which is in connection with the substance taken up by the hollow organ. The porosity at said above described surface of the fleece material is selected to the effect that the connective tissue proliferating from the hollow organ cannot penetrate the fleece material layer. Thereby, the risk that the hollow organ might become clogged or overgrown later on, is minimized.

According to a further embodiment, the fleece material layer comprises an outer surface and an inner structure which renders possible or facilitates the integration of the connective tissue.

Finally, it is of advantage if the fleece material layer is tightly connected with the support body of the vascular support. This is suitable primarily because, in this manner, the position of the fleece material layer relative to the support body will not be changed during the implanting of the vascular support as well as subsequently, i.e. *in situ.*

According to a further embodiment, it is provided that the elasticity and the porosity of the fleece material are adjusted in such a manner that the desired pore size for promoting a well-aimed cell migration will be obtained only after a possible intended dilatation of the vascular support. The biostable fleece material will expand corresponding to the dilatation of the support body, as far as such a preferably permanently expandable support body is used in the inventive vascular support. In such a vascular support, it is of advantage if, in the dilated state, the pore size of the fleece material has the desired value or is in the desired range of values. On the basis of the degree of dilatation and the properties of the fleece material used, it can be determined, by backward calculation, which pore size the fleece material should have in the not-yet-dilated state of the support body in order to accomplish a desired pore size or range of pore sizes in the fleece material and respectively in its surfaces. In any case, the elasticity of the fleece material has to be provided to the effect that the dilated support body cannot be squeezed together again by the widened fleece material.

The arrangement of a layer of biostable fleece material with at least partial abutment on the support body of a vascular support surprisingly leads to a lower postoperative complication rate after implantation of a vascular support for the purpose of vessel dilatation. Both the risk of plaque rupture and the consequences of such a plaque rupture due to the placement of a vascular support are considerably reduced by using a fleece material layer on the support body. In this manner, an occlusion of peripheral vessels is prevented.

According to a further embodiment, it is provided that the biostable fleece material completely covers the outer side and/or the inner side of the support body.

A further functional advantage of the invention can be seen in the feature that the enclosure does not represent a compactly closed structure but instead consists of a three-dimensional, microporous, fine-fibrillated fiber structure. Thereby, the physiology of the vessel wall is not restricted as much as when using dense, closed materials. Thus, this material structure allows for an exchange of substances from and to the vessel wall and also offers the possibility of a selective adhesion, migration and proliferation of cells. The result is the generation of an endothelium-like layer which can be formed toward the blood.

By the differentiated configuration of the fleece structure, a well-aimed colonization of cells is achieved. Thereby, the vascular support will become completely fixed in position by integrative healing, so that the inventive product can be conceived of as a catalyst for the reestablishment of physiological conditions on the vessel wall.

The invention described herein is applicable in biological vessel systems, particularly in coronary vessels, peripheral vessels (arterial and venous applications) and neurovascular vessels. Apart from these vessels, the hollow organs which can be kept open by the inventive vascular support also include lymph vessels, renal ducts, urethrae, the esophagus, nerve cords or uterine tubes.

In another application, the enclosed placement body as provided according to the invention can be used for therapy of vessel aneurysms of any type (fusiform, sacriform, pedunculated and non-pedunculated aneurysms). By the presence of the microporous fleece structure, there will first occur a stasis and a thrombosis formation in the aneurysmal sac. Subsequent wound healing processes with an absorption of the thrombus and a replacement of connective tissue will allow the aneurysm to heal. Also here, the later formation of a functional endothelium layer on the inner side of the implant will very quickly lead to laminar flow conditions in the area of the aneurysm. This physiological replacement for closure of the aneurysmal sac is safer and requires distinctly less time for the surgical intervention than is possible e.g. through the conservative method by filling with coils. By implanting the placement body the aneurysmal sac is bypassed so that blood flows through the lumen of the placement body, thereby preventing further ingress of blood into the aneurismal sac. Moreover, this closure of the aneurysmal sac prevents thrombogenesis in the blood vessel.

According to a still further embodiment, the invention is suited for use also in the non-vascular region, e.g. as a gastrointestinal stent. Also in this application, the promotion of a physiological cell proliferation by the fine-fibrillated fleece for thus forming a natural vessel-wall layer is of eminent advantage.

In case of an application for tumor diseases, the tumor tissue can hardly grow through the areal enclosure into the lumen.

The biostable fleece material of the device of the invention is suitably formed from an elastomer, preferably form a thermoplastic elastomer. With preference, the fleece structure is made of polyurethane, particularly linear polyurethane. With particular advantage, the polyurethane is an aliphatic polyurethane, preferably formed of macromolecular and/or low-molecular aliphatic diols as well as aliphatic diisocyanates. According to the invention, it is especially preferred that said macromolecular diols are polycarbonates, particularly 1,6-hexanediol polycarbonate. Said low-molecular diols preferably are 2,2,4-trimethylhexanediol, 2,4,4-trimethylhexanediol and/or 1,4-butanediol. Preferably, said aliphatic diisocyanates are 4,4'-dicyclohexylmethane diisocyanate or 1,4-cyclohexyl diisocyanate. According to the invention, it can further be preferred that said aliphatic polyurethane is formed of different diols and/or diisocyanates, wherein preference is given to the diols and diisocyanates described in this paragraph. Concerning further details and features of polyurethanes, reference is made to DE-A-36 43 465, DE-A-33 18 730, DE-A-41 07 284 and to the polymer report "Biocompatible Polyurethanes for Medical Techniques" of the research institute of Enka AG in Obernburg.

According to the invention, the fleece material layer comprises fibers having a diameter from 0.1 µm to 100 µm, preferably from 0.2 µm to 20 µm and more preferably from 0.3 µm to 1 µm.

According to the arrangement of the invention, the fleece material layer has a bottom side which is in abutment on the outer side of the support body, and a top side facing away from the outer side of the support body. Further, the fleece material layer comprises, on its top side, pores of a size different from that of the pores on its bottom side. According to the invention, the pores on the bottom side of the fleece material layer are smaller than the pores on the top side of the fleece material layer. The ratio between the pore size on bottom side and the pore size on top side may be 1:50, preferably 2:10 and more preferably 4:8.

The fleece material layer may have a thickness from 10 µm to 3000 µm.

According to a preferred embodiment of the invention, the support body is expandable, particularly in a permanent manner, with the fleece material layer being stretched at the same time, wherein, prior to the expansion, the thickness of the fleece material layer is from 100 µm to 3000 µm, preferably from 150 µm to 2800 µm and more preferably between 200 µm and 2000 µm.

According to a further preferred embodiment of the invention, the support body is expandable, particularly in a permanent manner, with the fleece material layer being stretched at the same time, wherein, after the expansion, the thickness of the fleece material layer is from 10 µm to 2500 µm, preferably from 20 µm to 2000 µm and more preferably between 80 µm and 1000 µm.

According to embodiments, the vascular support comprises a further layer of biostable fleece material, wherein the inner side and the outer side of the support body each comprise respectively one fleece material layer which is arranged at least partially in abutment on the respective side.

According to embodiments, the support body is porous and particularly has a reticular structure.

In the inventive method for producing the vascular support, there can be performed e.g. the procedural steps described in the Applicant's PCT Patent Application PCT/EP2010/066928. According to these methods, the fleece material is sprayed in the form of microfibers onto a rotating shaped member. According to the invention, said shaped member may comprises the support body of the vascular support.
According to said method, there is first provided the support body and, with the aid of a spraying device, the support body is sprayed with fibers for thus applying the layer of biostable fleece material. In the process, the support body and the spraying device are moved relative to each other. In dependence on the desired thickness of the layer of biostable fleece material and/or the desired porosity, a plurality of fiber layers will be spray-deposited, optionally with different areal densities.

A full and enabling disclosure of the present invention, including the best mode thereof, enabling one of ordinary skill in the art to carry out the invention, is set forth in greater detail in the following description, including reference to the accompanying drawing in which
- Fig. 1: is a lateral view of a tubular vascular support to be used in a blood vessel for holding open the blood vessel, wherein a part of the illustrated vascular support is broken away to visualize the wall structure of the vascular support, and
- Fig. 2: is an enlarged view of the detail II in Fig. 1.

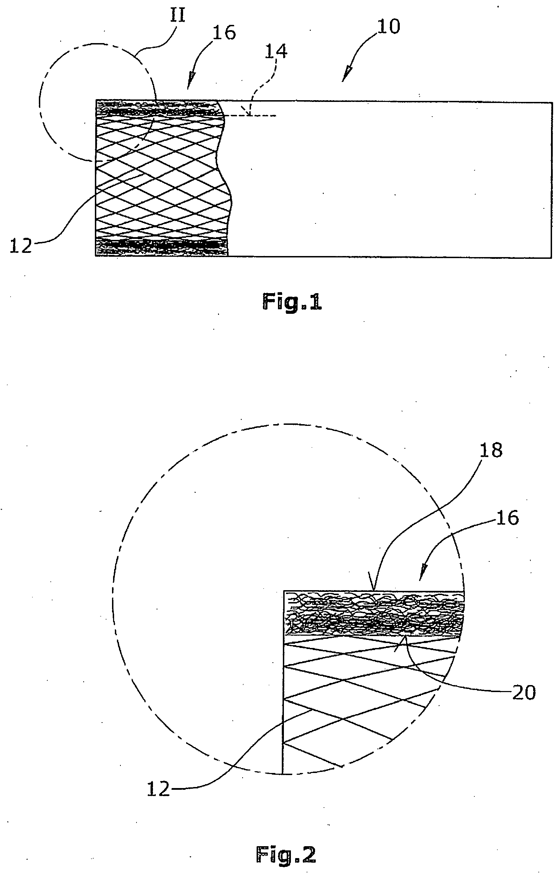

Fig. 1 shows, in lateral view, a tubular vascular support 10 (e.g. a stent) comprising a reticular or net-shaped support body 12 whose cylindrical outer side 14 is provided with an enclosure made of a layer 16 of biostable fleece material. Said fleece material layer 16 comprises a random-laid fleece made of microfibers. Fleece material layer 16 has a larger porosity on its outer side 18 than on its inner side 20. By its outer side 18, fleece material layer 16 is arranged in abutment on the vessel wall (not shown). As achieved by the invention, tissue proliferating from the vessel wall will only partially intrude into the fleece material layer 16. Such a proliferation of the tissue will be stopped at the latest in that region of the fleece material layer 16 which is located near the support body 12, particularly on the inner side 20 of layer 16 whose pore size is selected to the effect that a further proliferation of tissue through the fleece material layer 16 will not be possible anymore.

## Claims

1. A device for placement in a hollow organ, in particular for holding open said hollow organ, comprising:
- a placement body (12) having an inner side and an outer side (14), and
- at least one layer (16) of biostable random-fiber fleece material arranged at said placement body (12) and at least partially in abutment thereon, wherein
said random-fiber fleece material layer (16) is at least partially arranged in abutment on said outer side (14) of the placement body (12), **characterized in that** the random-fiber fleece material layer (16) has a bottom side which is in abutment on the outer side (14) of the placement body (12), and a top side facing away from the outer side (14) of the placement body (12), wherein the random-fiber fleece material layer (16) on its top side comprises pores of a size different from that of the pores on its bottom side, and wherein the pores on the bottom side of the random-fiber fleece material layer (16) are smaller than the pores on the top side of the random-fiber fleece material layer (16).

2. The device according to claim 1, wherein the random-fiber fleece material layer (16) comprises fibers having a diameter from 0.1 µm to 100 µm, preferably from 0.2 µm to 20 µm and more preferably from 0.3 µm to 1 µm.

3. The device according to claim 1 or 2, wherein the ratio between the pore size on bottom side and the pore size on top side is 1:50, preferably 2:10 and more preferably 4:8.

4. The device according to any one of claims 1 to 3, wherein the random-fiber fleece material layer (16) has a thickness from 10 µm to 3000 µm.

5. The device according to any one of claims 1 to 4, wherein the placement body (12) is particularly permanently expandable, with the random-fiber fleece material layer (16) being stretched at the same time, and wherein, prior to expansion, the thickness of the random-fiber fleece material (16) layer is from 100 µm to 3000 µm, preferably from 150 µm to 2800 µm and more preferably between 200 µm and 2000 µm.

6. The device according to any one of claims 1 to 5, wherein the placement body (12) is particularly permanently expandable, with the random-fiber fleece material layer (16) being stretched at the same time, and wherein, after expansion, the thickness of the random-fiber fleece material layer (16) is from 10 µm to 2500 µm, preferably from 20 µm to 2000 µm and more preferably between 80 µm and 1000 µm.

7. The device according to any one of claims 1 to 6, wherein a further layer of biostable random-fiber fleece material is provided, the inner side and the outer side of the placement body (12) each comprising respectively one random-fiber fleece material layer arranged at least partially in abutment thereon.

8. The device according to any one of claims 1 to 7, wherein the placement body (12) is porous and particularly has a reticular structure.

9. A method for producing a device according to any one of the preceding claims, wherein the placement body (12) is provided and, with the aid of a spraying device, the placement body (12) is sprayed with fibers for thus applying said layer (16) of biostable random-fiber fleece material while the placement body (12) and the spraying device are moved relative to each other, and wherein, in dependence on the desired thickness of the layer (6) of biostable random-fiber fleece material, a plurality of fiber layers are spray-deposited, and said random-fiber fleece material layer (16) is at least partially arranged in abutment on an outer side (14) of the placement body (12), **characterized by** arranging said random-fiber fleece material layer (16) with a bottom side in abutment on the outer side (14) of the placement body (12), and with a top side facing away from the outer side (14) of the placement body (12), and wherein the random-fiber fleece material layer (16) is arranged on its top side to comprise pores of a size different from that of the pores on its bottom side, and such that the pores on the bottom side of the random-fiber fleece material layer (16) are smaller than the pores on the top side of the random-fiber fleece material layer (16).

## Patentansprüche

1. Vorrichtung zur Platzierung in einem hohlen Organ, insbesondere zum Offenhalten des hohlen Organs, umfassend:
- Platzierungskörper (12), der eine Innenseite und eine Außenseite (14) aufweist, und
- mindestens eine Schicht (16) aus einem biostabilen Wirrfaservlies-Material, die an dem Platzierungskörper (12) und mindesten teilweise in Anlage auf diesem angeordnet ist, wobei
die Wirrfaservlies-Materialschicht (16) mindestens teilweise in Anlage auf der Außenseite (14) des Platzierungskörpers (12) angeordnet ist, **dadurch gekennzeichnet, dass** die Wirrfaservlies-Materialschicht (16) eine Unterseite, die sich in Anlage auf der Außenseite (14) des Platzierungskörpers (12) befindet, und eine Oberseite aufweist, welche von der Außenseite (14) des Platzierungskörpers (12) weg weist, wobei die Wirrfaservlies-Materialschicht (16) auf ihrer Oberseite Poren einer Größe umfasst, welche sich von jener der Poren auf ihrer Unterseite unterscheidet, und wobei die Poren auf der Unterseite der Wirrfaservlies-Materialschicht (16) kleiner sind als die Poren auf der Oberseite der Wirrfaservlies-Materialschicht (16)

2. Vorrichtung gemäß Anspruch 1, wobei die Wirrfaservlies-Materialschicht (16) Fasern umfasst, welche einen Durchmesser von 0.1 µm bis 100 µm, bevorzugt von 0.2 µm bis 20 µm und noch stärker bevorzugt von 0.3 µm bis 1 µm aufweisen.

3. Vorrichtung gemäß Anspruch 1 oder 2, wobei das Verhältnis zwischen der Porengröße auf der Unterseite und der Porengröße auf der Oberseite 1:50, bevorzugt 2:10 und noch stärker bevorzugt 4:8 beträgt.

4. Vorrichtung einem der Ansprüche 1 bis 3, wobei die Wirrfaservlies-Materialschicht (16) eine Dicke von 10 µm bis 3000 µm aufweist.

5. Vorrichtung gemäß einem der Ansprüche 1 bis 4, wobei der Platzierungskörper (12) insbesondere dauerhaft ausdehnbar ist, wobei die Wirrfaservlies-Materialschicht (16) gleichzeitig gedehnt wird, und wobei die Dicke der Wirrfaservlies-Materialschicht (16) vor der Ausdehnung von 100 µm bis 3000 µm, bevorzugt von 150 µm ist 2800 µm und noch stärker bevorzugt zwischen 200 µm und 2000 µm beträgt.

6. Vorrichtung gemäß einem der Ansprüche 1 bis 5, wobei der Platzierungskörper (12) insbesondere dauerhaft ausdehnbar ist, wobei die Wirrfaservlies-Materialschicht (16) gleichzeitig gedehnt wird, und wobei die Dicke der Wirrfaservlies-Materialschicht (16) nach der Ausdehnung von 10 µm bis 2500 µm, bevorzugt von 20 µm bis 2000 µm und noch stärker bevorzugt zwischen 80 µm und 1000 µm beträgt.

7. Vorrichtung gemäß einem der Ansprüche 1 bis 6, wobei eine weitere Schicht aus biostabilem Wirrfaservlies-Material vorgesehen wird, wobei die Innenseite und die Außenseite des Platzierungskörpers (12) jeweils eine Wirrfaservlies-Materialschicht umfassen, die mindesteins teilweise in Anlage an diese angeordnet ist.

8. Vorrichtung gemäß einem der Ansprüche 1 bis 7, wobei der Platzierungskörper (12) porös ist und insbesondere eine retikuläre Struktur aufweist.

9. Verfahren zur Herstellung einer Vorrichtung gemäß einem der vorangehenden Ansprüche, wobei der Platzierungskörper (12) vorgesehen ist und der Platzierungskörper (12) mithilfe einer Sprühvorrichtung mit Fasern besprüht wird, um auf diese Weise die Schicht (16) aus biostabilem Wirrfaservlies-Material aufzutragen, während der Platzierungskörper (12) und die Sprühvorrichtung in Bezug aufeinander bewegt werden, und wobei in Abhängigkeit von der gewünschten Dicke der Schicht (16) aus biostabilem Wirrfaservlies-Material eine Vielzahl von Faserschichten sprühbeschichtet wird, und die Wirrfaservlies-Materialschicht (16) mindestens teilweise in Anlage auf die Außenseite (14) des Platzierungskörpers (12) angeordnet ist, **dadurch gekennzeichnet, dass** die Wirrfaservlies-Materialschicht (16) mit einer Unterseite in Anlage an die Außenseite (14) des Platzierungskörpers (12) und mit einer Oberseite von der Außenseite (14) des Platzierungskörpers (12) weg weisend angeordnet wird, und wobei die Wirrfaservlies-Materialschicht (16) derart angeordnet ist, dass sie auf ihrer Oberseite Poren einer Größe umfasst, die sich von jener der Poren auf ihrer Unterseite unterscheidet, und zwar derart, dass die Poren auf der Unterseite der Wirrfaservlies-Materialschicht (16) kleiner sind als die Poren auf der Oberseite der Wirrfaservlies-Materialschicht (16).

## Revendications

1. Dispositif pour placement dans un organe creux, en particulier pour maintenir ouvert ledit organe creux, comprenant :
- un corps de placement (12) ayant un côté intérieur et un côté extérieur (14), et
- au moins une couche (16) d'un tissu molletonné à fibres aléatoires biostable agencé au niveau dudit corps de placement (12) et au moins en partie en butée contre celui-ci, où
ladite couche de tissu molletonné à fibres aléatoires (16) est au moins partiellement agencée en butée contre ledit côté extérieur (14) du corps de placement (12), **caractérisé en ce que** la couche de tissu molletonné à fibres aléatoires (16) présente un côté inférieur lequel vient en butée contre le côté extérieur (14) du corps de placement (12), et un côté supérieur orienté vers l'opposé du côté extérieur (14) du corps de placement (12), où la couche de tissu molletonné à fibres aléatoires (16) sur son côté supérieur comprend des pores de taille différente de celle des pores sur son côté inférieur, et où les pores sur le côté inférieur de la couche de tissu molletonné à fibres aléatoires (16) sont plus petits que les pores sur le côté supérieur de la couche de tissu molletonné à fibres aléatoires (16).

2. Dispositif selon la revendication 1, dans lequel la couche de tissu molletonné à fibres aléatoires (16) comprend des fibres de diamètre compris entre 0,1 µm et 100 µm, de préférence entre 0,2 µm et 20 µm, et de façon davantage préférée entre 0,3 µm et 1 µm.

3. Dispositif selon la revendication 1 ou 2, dans lequel le rapport entre la taille de pore sur le côté inférieur et la taille de pore sur le côté supérieur est de 1:50, de préférence de 2:10, et de façon davantage préférée de 4:8.

4. Dispositif selon l'une quelconque des revendications 1 à 3, dans lequel la couche de tissu molletonné à fibres aléatoires (16) a une épaisseur comprise entre 10 µm et 3 000 µm.

5. Dispositif selon l'une quelconque des revendications 1 à 4, dans lequel le corps de placement (12) peut en particulier se déployer de manière permanente, la couche de tissu molletonné à fibres aléatoires (16) étant étirée en même temps, et où, avant le déploiement, l'épaisseur de la couche de tissu molletonné à fibres aléatoires (16) est comprise entre 100 µm et 3 000 µm, de préférence entre 150 µm et 2 800 µm, et de façon davantage préférée entre 200 µm et 2 000 µm.

6. Dispositif selon l'une quelconque des revendications 1 à 5, dans lequel le corps de placement (12) peut en particulier se déployer de manière permanente, la couche de tissu molletonné à fibres aléatoires (16) étant étirée en même temps, et où, avant le déploiement, l'épaisseur de la couche de tissu molletonné à fibres aléatoires (16) est comprise entre 10 µm et 2 500 µm, de préférence entre 20 µm et 2 000 µm, et de façon davantage préférée entre 80 µm et 1 000 µm.

7. Dispositif selon l'une quelconque des revendications 1 à 6, dans lequel une autre couche de tissu molletonné à fibres aléatoires biostable est prévue, le côté intérieur et le côté extérieur du corps de placement (12) comprenant chacun respectivement une couche de tissu molletonné à fibres aléatoires agencée au moins partiellement en butée contre ledit côté.

8. Dispositif selon l'une quelconque des revendications 1 à 7, dans lequel le corps de placement (12) est poreux et présente en particulier une structure réticulaire.

9. Procédé pour produire un dispositif selon l'une quelconque des revendications précédentes, dans lequel le corps de placement (12) est fourni et, à l'aide d'un dispositif de pulvérisation, le corps de placement (12) subit une pulvérisation de fibres pour recevoir ainsi ladite couche (16) de tissu molletonné à fibres aléatoires biostable, tandis que le corps de placement (12) et le dispositif de pulvérisation sont déplacés l'un par rapport à l'autre, et où, en fonction de l'épaisseur voulue de la couche (16) de tissu molletonné à fibres aléatoires biostable, une pluralité de couches de fibres sont déposées par pulvérisation, et ladite couche de tissu molletonné à fibres aléatoires (16) est au moins partiellement agencée en butée contre un côté extérieur (14) du corps de placement (12), **caractérisé par le fait que** ladite couche de tissu molletonné à fibres aléatoires (16) est agencée de sorte à présenter un côté inférieur en butée contre le côté extérieur (14) du corps de placement (12) et un côté supérieur orienté vers l'opposé du côté extérieur (14) du corps de placement (12), et où la couche de tissu molletonné à fibres aléatoires (16) est agencée sur son côté supérieur pour comprendre des pores de taille différente de celle des pores sur son côté inférieur, et de telle sorte que les pores sur le côté inférieur de la couche de tissu molletonné à fibres aléatoires (16) sont plus petits que les pores sur le côté supérieur de la couche de tissu molletonné à fibres aléatoires (16).
